# EUROPEAN PATENT APPLICATION

(11) **EP 4 541 881 A1**
(43) Date of publication of application: **23.04.2025**
(21) Application number: 23826322.2
(22) Date of filing: 19.06.2023
(51) Int. Cl.: C12N 1/20, A23L 33/135, A61K 35/745, A61P 3/00, A61P 3/04, A61P 3/10, A61P 5/50, C12R 1/01

(54) **BIFIDOBACTERIUM BIFIDUM FOR TREATING DIABETES AND RELATED CONDITIONS**

(30) Priority: 21.06.2022 CN 202210704856; 11.08.2022 CN 202210962600
(71) Applicant: IBIOME Biotechnology Co., Ltd., Hefei, Anhui 230601 (CN)
(72) Inventor: ZHU, Shu, Hefei, Anhui 230601 (CN); WEN, Wen, Hefei, Anhui 230601 (CN); SUN, Shanshan, Hefei, Anhui 230601 (CN); TAO, Wanyin, Hefei, Anhui 230601 (CN)
(74) Representative: Dantz, Jan Henning
(86) International application number: PCT/CN2023/100940
(87) International publication number: WO 2023/246674

(57) **Abstract**

Provided is a *Bifidobacterium bifidum* strain, *Bifidobacterium bifidum* ibiome001, which can significantly reduce body weight gain and white adipose tissue weight gain of high fat diet-induced obese and diabetic mice, and enhances expression of lipolytic genes ATGL and HSL. Moreover, *Bifidobacterium bifidum* ibiome001 can significantly lower fasting blood glucose levels, area under curves (AUCs) of the oral glucose tolerance (OGTT) curve, plasma glycated hemoglobin levels and insulin levels of diabetic mice. It is suggested that *Bifidobacterium bifidum* ibiome001 is a promising potential probiotic strain for the treatment of metabolic syndrome such as obesity, diabetes and the like, which is more effective than other *Bifidobacterium* strains/compositions.

## Description

### Technical Field

The invention relates to the technical field of microorganism, specifically to Bifidobacterium bifidum for treating diabetes mellitus and related diseases.

### Background Art

With the development of society, the continuous improvement of people's living standards and the adjustment of diet structure, the diet shows a trend of excess and rich, and high-fat diet has become an important way of daily diet. However, this has led to increasing incidence of metabolic diseases, among which metabolic syndrome such as obesity and diabetes is the most common, which seriously affects people's physical and mental health.

There are about 1.5 kg of bacteria in the gut. Through long-term co-evolution with the host, the vast number of intestinal florae has formed an inseparable structural and functional relationship. Gut microbiota participates in the host homeostasis by digesting nutrients, providing host vitamins and energy, and by participating in normal immune construction. It has been considered that dysbiosis of intestinal flora has been linked to more than 50 diseases, and recent studies have found that metabolic diseases, especially obesity and diabetes, are closely related to gut microbiota, which can regulate host fat accumulation and insulin sensitivity. Clinical FMT research showed significant improvement in insulin resistance in obese patients after receiving 6-week fecal microbiota transplantation (FMT) from healthy donors; and another study identified 52,484 enterobacterial genes associated with Type 2 Diabetes Mellitus(T2DM) in 171 Chinese adult T2DM patients and 174 healthy volunteers. Therefore, it has been suggested that probiotics have great potential in treating obesity and diabetes.

GPR120 is a long-chain unsaturated free fatty acid receptor, which has multiple physiological functions such as regulating gastrointestinal hormone secretion and regulating development and differentiation of adipocytes. Animal experiments showed that high-fat diet induced GPR120-null mice developed more severe obesity, insulin resistance and liver steatosis, and a series of pre-clinical studies showed that GPR120 agonists can regulate glucose and energy homeostasis, including improving obesity-induced chronic inflammation and insulin resistance, regulating adipocyte thermogenesis, and regulating appetite. Moreover, a human cohort study also showed a significant association of R270H mutation in GPR120L amino acid sequence with obesity. Thus, GPR120 has been considered as a potentially important target for treating metabolic syndromes such as obesity, diabetes, etc.

About 95% of total triglyceride (TG) is degraded by the two types of triglyceride lipases in adipose tissue, adipose triglyceride lipase (ATGL) and hormone-sensitive lipase (HSL). ATGL is active without hormonal activation, which is very important in basal lipolysis, and is also the most important lipolytic enzyme, while HSL requires activation by lipolytic hormones for its activity.

HSL was discovered in 1962 and got its name because its lipase activity is strongly affected by hormones. It has been shown that HSL activators can phosphorylate HSL via PKA to trans-localize HSL into lipid droplets, thereby facilitating the lipolysis process, and insulin is the most important inhibitor of HSL.

ATGL was discovered in 2004. The C-terminus of ATGL contains a hydrophobic lipid droplet binding region, so it is mainly localized on the surface of lipid droplets. ATGL can specifically hydrolyze the first ester bond of TG, and is considered to be the rate-limiting enzyme in TG hydrolysis process. Reduction of ATGL gene expression can lead to a large accumulation of TG in adipocytes and other tissues, resulting in obesity and other metabolic complications.

### SUMMARY OF THE INVENTION

### Technical Problems

It is an object of the present invention to provide a probiotic *Bifidobacterium bifidum* strain for the treatment of diabetes and related conditions.

### Means to solve the Problem

The present invention is implemented by the following technical solution:

*Bifidobacterium bifidum* ibiome001, the *Bifidobacterium bifidum* strain was deposited at Guangdong Microorganism Strain Preservation Center, the address is the 5th floor, Building 59, No. 100, Xianlie Central Road, Guangzhou City, Guangdong Province, Institute of Microbiology, Guangdong Academy of Sciences; the deposit date is May 17, 2022 and the accession number is GDMCC No. 62473. Its whole genome sequence is shown as SEQ ID NO.2.

As used herein, the term *"Bifidobacterium bifidum* ibiome001" means the *Bifidobacterium bifidum* strain whose genome contains at least one specific gene segment or complementary segment contained in SEQ ID NO.2-5.

The present invention also protects the use of the *Bifidobacterium bifidum* ibiome001 and metabolites, or mixtures containing the bacterium and/or its metabolites, in the preparation of functional microbial agents or pharmaceuticals, wherein the functional microbial agents or pharmaceuticals are used to prevent or treat one, two or more of the following diseases and symptoms (a) to (j) in mammals:
(a) diabetes;
(b) metabolic syndrome;
(c) abnormal glycated hemoglobin levels;
(d) abnormal insulin sensitivity;
(e) impaired fasting glucose;
(f) abnormal oral glucose tolerance;
(g) abnormal fasting insulin levels;
(h) obesity;
(i) body weight gain; and
(j) increased adipose tissue weight.

The present invention also protects the use of the *Bifidobacterium bifidum* ibiome001 and its metabolites, or mixtures containing the bacterium and/or its metabolites, in the preparation of functional microbial agents or pharmaceuticals, wherein the functional microbial agents or pharmaceuticals are used for activating GPR120.

The present invention also protects the use of the *Bifidobacterium bifidum* ibiome001 and its metabolites, or mixtures containing the bacterium and/or its metabolites, in the preparation of functional microbial agents or pharmaceuticals, wherein the functional microbial agents or pharmaceuticals are used for enhancing the expression of the lipolytic genes ATGL and/or HSL.

As used herein, the term "diabetes" means type 2 diabetes mellitus (T2DM).

As used herein, the term "mammal" means obese mammal models, wherein the model is high-fat diet-induced or spontaneous.

The present invention also protects the use of the *Bifidobacterium bifidum* ibiome001 and its metabolites, or mixtures containing the bacterium and/or its metabolites, in preparing food or dietary supplement.

The present invention also claims a composition comprising the *Bifidobacterium bifidum* ibiome001, or its metabolites, or mixtures containing the bacterium and/or its metabolites, wherein the composition contains at least one pharmaceutically acceptable carrier.

As used herein, "pharmaceutically acceptable carrier" may be defined as carriers commonly used in medication and pharmacy, including filling agent, binding agent, wetting agent, disintegrating agent, lubricant, flavoring agent, diluent agent, absorption promoting agent and the like.

### Advantage of the Invention

The beneficial effects of the invention are:

The present inventors have found that gavage of *Bifidobacterium bifidum* ibiome001 can significantly reduce body weight and white adipose tissue weight of high-fat diet-induced obese diabetic mice, and enhances the expression of the lipolytic gene ATGL and HSL. Moreover, fasting glucose, oral glucose tolerance, plasma glycated hemoglobin and insulin levels have been significantly improved in high-fat diet-induced obese and diabetic mice after receiving gavage of *Bifidobacterium bifidum* ibiome001, and gavage of *Bifidobacterium bifidum* ibiome001 shows the best improvement effect than other *Bifidobacterium bifidum* strains or *Bifidobacterium bifidum* compositions. Thus, *Bifidobacterium bifidum* ibiome001 may be considered as a potential functional strain for preventing and treating metabolic syndromes such as obesity, diabetes and the like.

### Reference To Deposited Biological Material

*Bifidobacterium bifidum* ibiome001, the deposit date is 17th May, 2022, the deposit location is at Guangdong Microorganism Strain Preservation Center, the address is 5th Floor, Building 59, No. 100, Xianlie Central Road, Guangzhou City, Guangdong Province, Institute of Microbiology, Guangdong Academy of Sciences, the deposit accession number is GDMCC No. 62473.

### BRIEF DESCRIPTION OF THE DRAWINGS

FIG. 1 shows a smear microscopic diagram (40×) of ibiome001 according to the present invention.
FIG. 2 shows the colony morphology of ibiome001 of the present invention on solid culture medium.
FIG. 3 shows the gene alignment map of ibiome001 according to the present invention with other *Bifidobacterium bifidum.*
FIGs. 4-7 show a magnified view of gene alignment of ibiome001 according to the present invention with other *Bifidobacterium bifidum* at SEQ ID NO. 2-5.
FIG. 8 shows the results of GPR120 activation by different strains of *Bifidobacterium bifidum.*
FIG. 9 shows the effects of ibiome001 according to the present invention and other three groups of controls on the body weight of high-fat diet-induced obese and diabetic mice. a: the body weight change rate of obese and diabetic mice; b: the body weight value of obese and diabetic mice; *: P < 0.05; **: P < 0.01.
FIG. 10 shows the effects of ibiome001 according to the present invention and other three groups of controls on blood glucose values of high-fat diet-induced obese and diabetic mice. *: P < 0.05.
FIG. 11 shows the effects of ibiome001 according to the present invention and other three groups of controls on high-fat diet-induced obesity and diabetic mice oral glucose tolerance (OGTT) and area under curve (AUC) of OGTT. *: P < 0.05.
FIG. 12 shows the effects of ibiome001 according to the present invention and other three groups of controls on fat content of high-fat diet-induced obese and diabetic mice. *: P < 0.05; **: P < 0.01.
FIG. 13 shows the effects of ibiome001 according to the present invention and other three groups of controls on the expression of lipolytic genes in white adipose tissue of high-fat diet-induced obese and diabetic mice. *: P<0.05.
FIG. 14 shows the effects of ibiome001 according to the present invention and other three groups of controls on glycated hemoglobin levels of high-fat diet-induced obese and diabetic mice. ** :P<0.01.
FIG. 15 shows the effects of ibiome001 according to the present invention and other three groups of controls on plasma insulin levels of high-fat diet-induced obese and diabetic mice. *: P < 0.05; **: P < 0.01.

### DETAILED DESCRIPTION OF THE EMBODIMENTS

Hereinafter, the present invention will be described in further detail with reference to examples. It will be obvious to those skilled in the art that these examples are for illustrative purposes only and are not intended to the scope of the present invention. In addition, unless otherwise specified, methods without specific conditions or procedures are routine, and the reagents and materials used are commercially available.

### Example 1 Isolation and Identification of Strain

### 1 separation

10 fecal samples from healthy volunteers were stored in 20 vol% of glycerol in phosphate buffer saline (PBS), and the fecal samples were diluted in gradient to 10⁻⁵, 10⁻⁶ and 10⁻⁷ respectively.

Each diluent was spread to MRS broth plate (MRS broth medium is purchased from Beijing Solarbio Science & Technology Co., Ltd., Catalogue No. M8540) and cultured at 37°C for 48 hours in anaerobic chamber.

Pick a single colony to MRS broth liquid medium, then extract bacterial DNA from corresponding anaerobic cultures to prepare templates for PCR amplification with 16S rRNA universal primer (upstream primer 27F: AGAGTTTG ATCCTGGCTCAG, downstream primer 1492R: GGTTA CCTTGTTACGACTT).

### 2. Identification

### 2.1 16S rRNA sequencing

Amplified DNA samples are sent for sequencing, then identifies *Bifidobacterium bifidum* ibiome001 via *in vitro* screening by 16S rRNA gene sequence alignment selecting *Bifidobacterium bifidum.*

### Experimental Methods:

Pipette 100 µL of bacterial liquid culture, then centrifuge the culture for 2 min at a speed of 12000 rpm. Discard the supernatant, then add sterilized ddH₂O to re-suspend the precipitate to prepare template for PCR.

PCR reaction system: 10µL of 2×Taq Master Mix, 1µL of primer 1 (primer 341F), 1µL of primer 2 (primer 1492R), 6µL of ddH₂O, 2µL of bacterial liquid culture.

PCR procedure: 95°C for 3 min, 95°C for 15 s, 58°C for 15 s and 72°C for 30 s, repeat steps 2-4 for 35×, and 72°C for 5 min.

Amplified PCR product is sequenced and aligned with 16S rRNA genes in databases, and the result is shown as SEQ ID NO.1.

### 2.2 smear microscopy

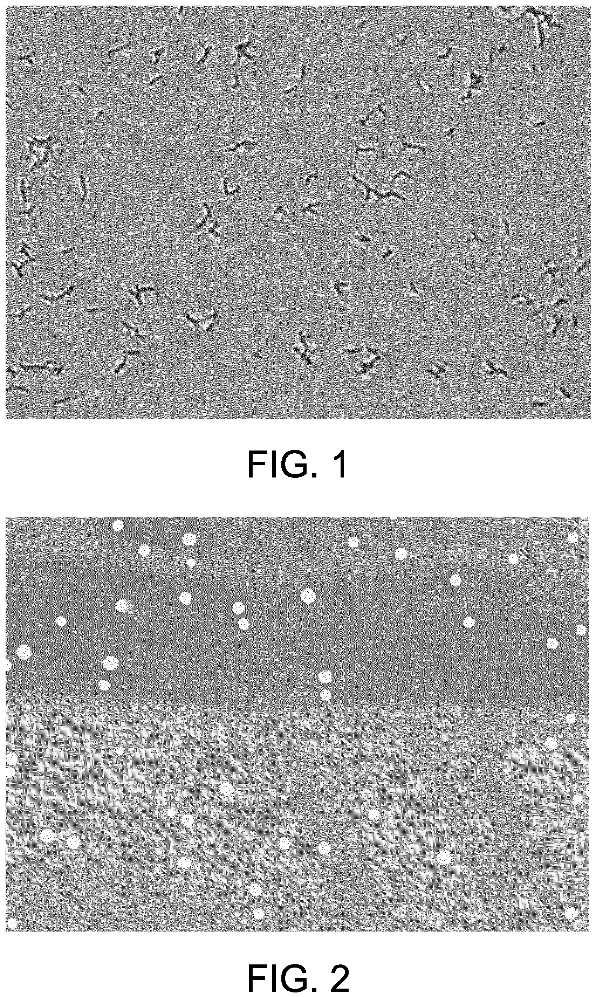

Observe ibiome001 culture smear under 40× objective lens, and the result is shown as FIG. 1. It has been shown that ibiome001 is a kind of nonmotile Gram-positive spore-less bacterium, with a shape of short-rod, slender-rod or spherical, and is able to form a variety of branches, forks or other morphologies.

### 2.3 Photographs of single colonies

The ibiome001 culture was spread on MRS plate, and the plate was photographed after a 48h-culture at 37°C in anaerobic chamber. The picture captured was shown as FIG. 2, and it has been shown that the colonies of ibiome001 were white and round with neat edges and a moist surface.

### 2.4 Detection of catalase test and sugar alcohol fermentation biochemical reaction

Thaw and pipette 300µL of ibiome001 culture, then add ibiome001 culture into 1mL MRS culture medium. After resuscitation, spread the liquid culture of ibiome001 on MRS broth plate, then pick single colony into 1 mL MRS liquid medium for a 24h-culture in anaerobic chamber. Gradient diluents of the liquid culture are spread on corresponding MRS broth plate and cultured for 72h in anaerobic chamber. Catalase assay and sugar alcohol fermentation assay is performed after anaerobic culture.

Catalase assay: Add 2-3 drops of 3% catalase reagent (purchased from Hope Bio-Technology Co., Ltd., catalogue number HB8650) onto the ibiome001 colonies. The result showed that there were no bubbles escaping from the surfaces of colonies, i.e., catalase-negative.

Sugar alcohol fermentation assay: Pick single colony of the ibiome001 culture and add to micro-biochemical identification tubes for bacteria (purchased from Hope Bio-Technology Co., Ltd., the catalogue numbers are GB057, GB102-1, GB104-1, GB176, GB178, GB188, GB189, GB193, GB195, GB196, GB197, GB199, GB200, GB201, GB202, GB203, GB204, GB206, GB207) with sterilized pipette tips, then culture the tubes at 37°C for 48h in anaerobic chamber. Identification results were recorded under the instruction of micro-biochemical identification tubes, as shown in Table 1:

**Table 1. Bifidobacterium bifidum biochemical reaction identification result**

| [Table 1_sm_0001] | | | |
|---|---|---|---|
| Detection item | Detection result | Detection item | Detection result |
| cellobiose | - | dulcitol | - |
| Trehalose | - | D-glucose | + |
| melibiose | - | D-galactose | + |
| inositol | - | Adonitol | - |
| D-maltose | - | amygdalin | - |
| L-arabinose | - | D-fructose | + |
| Salicin | - | glycogen | - |
| D-xylose | - | inulin | + |
| D-sucrose | - | N-acetyl-glucosa-mine | + |
| esculin | - | | |

| | | | |
|---|---|---|---|
| Note: + represents positive; - represents negative | | | |

### 2.5 whole genome sequencing

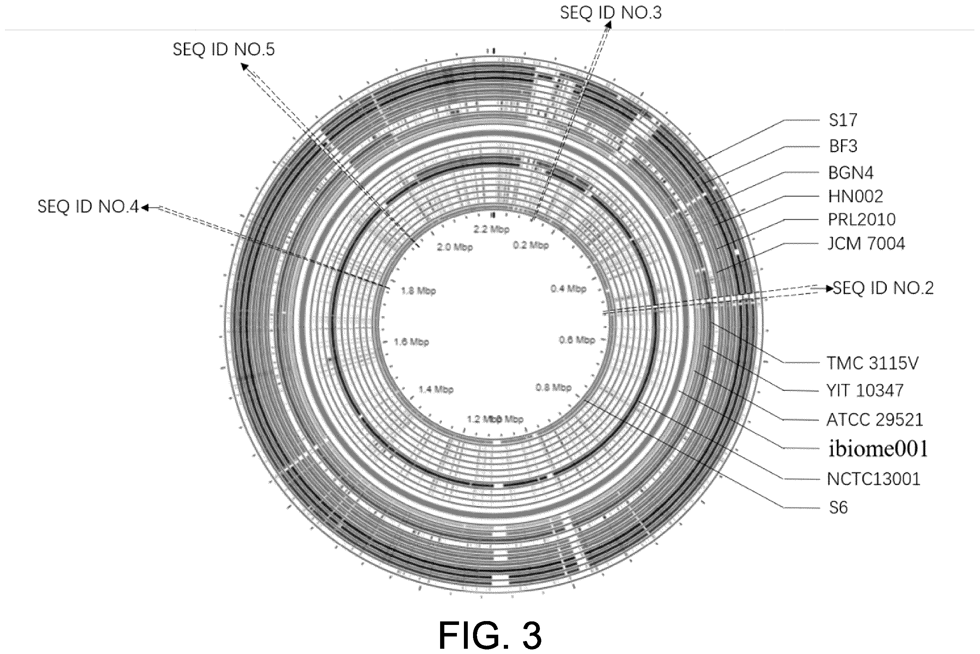

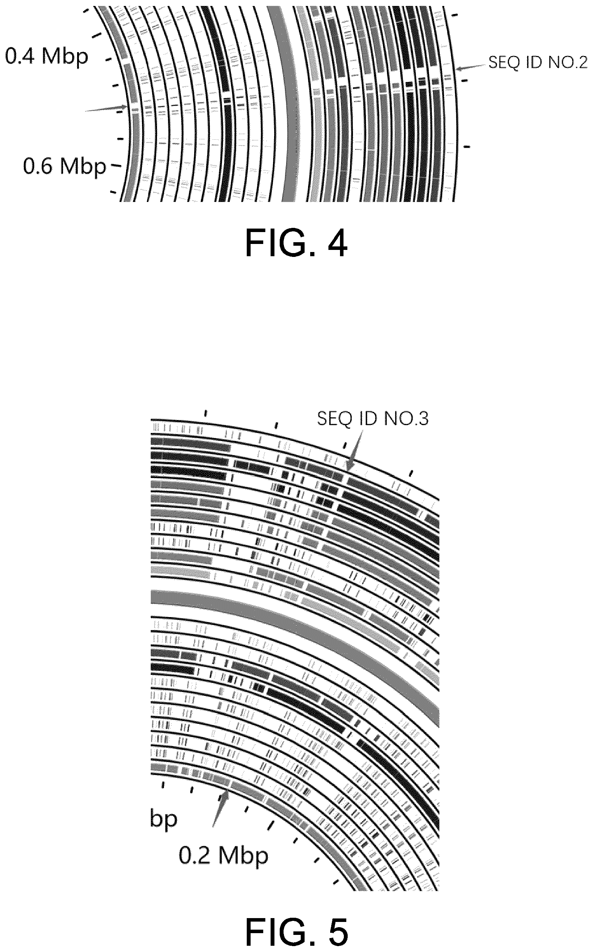

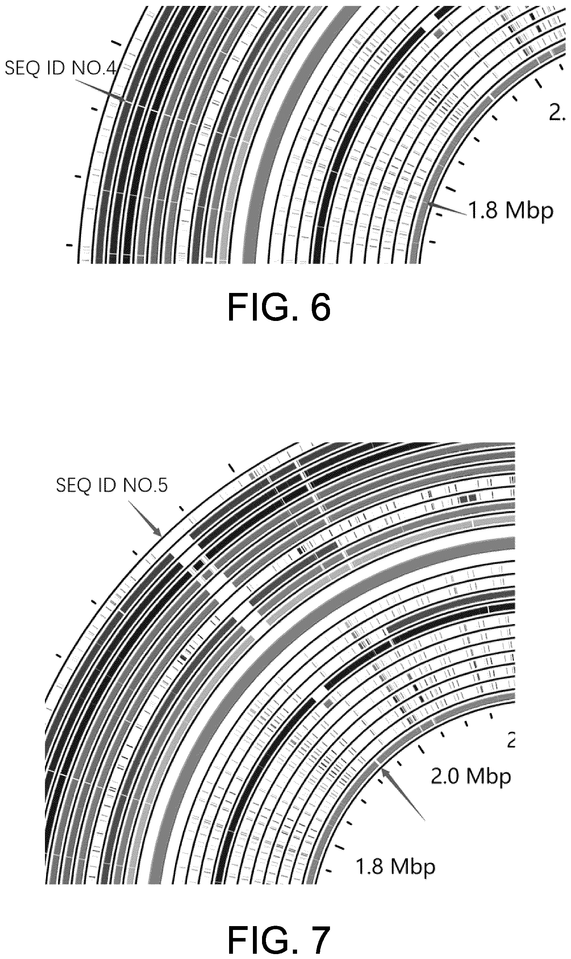

The genome of ibiome001 was sent to gene-sequencing company for whole genome sequencing, and the whole genome sequence was aligned with ATCC 29521 (=JCM 1255, GenBank Assembly Accession: GCA 001025135.1), YIT 10347 (GenBank Assembly Accession: GCA 020892075.1), TMC 3115 (GenBank Assembly Accession: GCA 003573895.1), NCTC13001 (GenBank Assembly Accession: GCA 900637095.1), JCM 7004 (GenBank Assembly Accession: GCA 003573955.1), PRL2010 (GenBank Assembly Accession: GCA-000165905.1), HN002 (GenBank Assembly Accession: GCA 016838705.1), BGN4 (GenBank Assembly Accession: GCA 000265095.1), BF3 (GenBank Assembly Accession: GCA 001281345.1), S17 (GenBank Assembly Accession: GCA 000164965.1), S6 (GenBank Assembly Accession: GCA 003390735.1) and genome sequences of other *Bifidobacterium bifidum* strains. The present inventors identified a specific gene segments of ibiome001, SEQ ID NO. 2-5, as shown in FIGs. 3-7.

### Example 2 Comparison of activation of GPR120 by different Bifidobacterium bifidum strains

(1) Culture the HEK293 cell strain, which was stable transfected by plasmids β-arrestin-TEV and tTA-luciferase, in DMEM culture medium containing 10 vol% fetal calf serum and 1 vol% Penicillin-Streptomycin;
(2) Transient transfection reagent preparation: for each well of cell culture, mix 200ng/well of GPR120-tango plasmid with 400ng of poly-ethylenimine (PEI) in 20µL of DMEM culture medium, then incubate the mixture at room temperature for 20 minutes;
(3) After the two-day culture of HEK293 cell (up to about 90 % fusion) according to step (1), add the transient transfection reagent prepared in step (2) into each well of the cell culture plate;
(4) After 24h of transfection, replace culture medium with 180µL of DMEM culture medium containing 1 vol% Penicillin-Streptomycin and 10mM HEPES, then add 20µL of supernatant of bacteria culture of different *Bifidobacterium bifidum* strains (Bb-1, Bb-2, Bb-3 or ibiome001, wherein Bb-1, Bb-2, Bb-3 are other *Bifidobacterium bifidum* strains containing consensus 16S rDNA sequences with SEQ ID NO.1 identified in laboratory);
(5) Discard the medium and add 50µL of Bright-Glo reagent (Promega Corporation) 20-fold diluted with PBS containing 20 mM HEPES to each well of cell culture;
(6) After incubating at room temperature for 20 minutes, quantify relative fluorescence intensity of each well with SpectraMax i3.

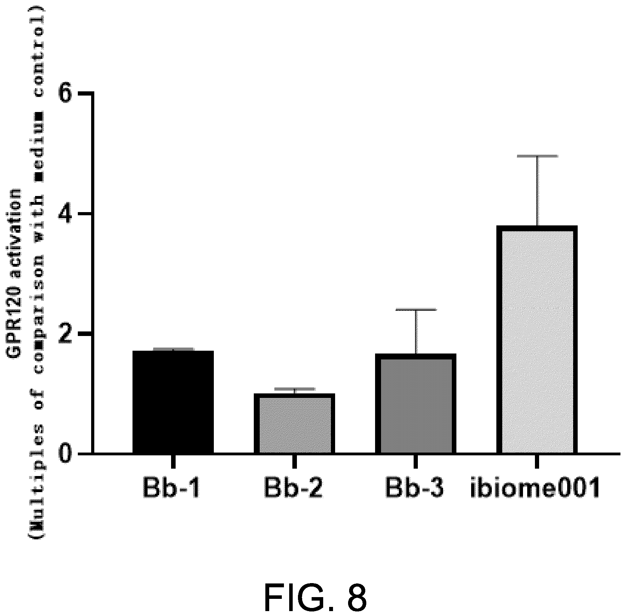

The results are shown in FIG. 8. It has been shown that ibiome001 induces the strongest activation of GPR120 gene expression, which is 4 times higher than medium control, while activation induced by other *Bifidobacterium bifidum* strains is less than 2 times higher than control. It is suggested that ibiome001 has the best ability to activate GPR120 expression than other *Bifidobacterium bifidum* strains.

### Example 3 Comparison of effects of different Bifidobacterium bifidum strains/compositions on mouse body weight, adipose tissue weight, lipolytic gene expression, oral glucose tolerance, fasting blood glucose, glycated hemoglobin levels and plasma insulin levels

C57BL/6J (10 weeks, male) mice, SPF grade, were purchased from GemPharmatech Co., Ltd., Jiangsu. After one-week adaptation, mice were given 60 % high fat diet (purchased from Medicience Ltd, Cat. No. MD12033). The experiments were divided into 4 groups:
(1) Control group: a control group, gavage of 0.2mL PBS saline;
(2) Bb group: gavage 10⁹ CFU of *Bifidobacterium bifidum* ibiome001 (Bb);
(3) Bb + BI group: gavage *Bifidobacterium bifidum* ibiome001 and *Bifidobacterium longum* (the bacterial cultures were mixed in the ratio of 1: 1; total number of colony is 10⁹ CFU);
(4) 5-mix (Ba + Bf + BI + Bb + Bp) group: five mixtures of *Bifidobacterium spp.,* which are *Bifidobacterium bifidum* ibiome001 (Bb), *Bifidobacterium longum* (BI), *Bifidobacterium adolescentis* (Ba), *Bifidobacterium faecalis* (Bf) and *Bifidobacterium pseudocatenum* (Bp), respectively (the bacterial cultures were mixed in the ratio of 1: 1: 1: 1: 1; total number of colony is 10⁹ CFU).

### 1. Effect on mouse body weight

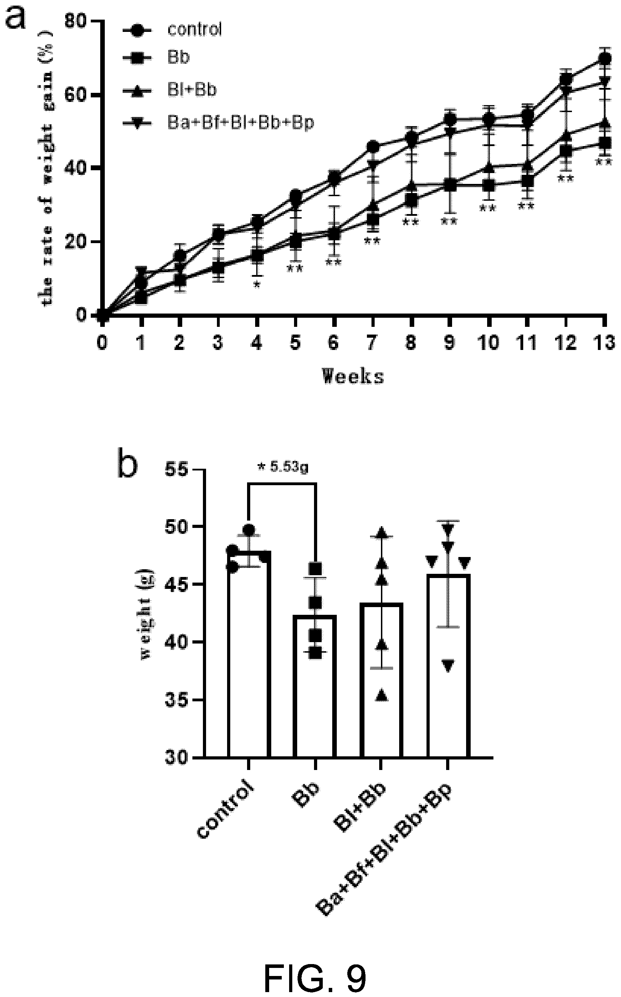

Before the start of the experiment, mice were divided into 4 groups as described above with similar average body weights, and body weights of mice were recorded weekly for 13 weeks. Relative and absolute body weight change of the mice are shown in FIG. 9.

It has been shown in a of FIG. 9 that gavage of *Bifidobacterium bifidum* ibiome001 (Bb) has significantly inhibited body weight gain of mice since week 4, while there is no significant inhibition of body weight gain in mice when given gavage of *Bifidobacterium bifidum* ibiome001 (Bb) composition containing other *Bifidobacterium spp.;* and it can be seen from b of FIG. 9 that the average body weight of Bb group mice was 5.53g lower than that of control after gavage of *Bifidobacterium bifidum* ibiome001 for 13 weeks.

### 2. Effect of mouse fasting blood glucose value and oral glucose tolerance

At week 10 of experiment, all groups of mice underwent fasting blood glucose tests and oral glucose tolerance tests (OGTT).

OGTT experimental procedure: before start, all groups of mice were fasted for 12 hours, and mouse fasting blood glucose levels were measured with glucometer and recorded as blood glucose at time point 0 of OGTT, then gavage glucose solution at a dosage of 2g/kg to the mouse successively. Blood glucose levels were measure and record at time points 30 min, 60 min and 120 min, and the blood glucose level-time curves were plotted and area under the curves (AUC) were calculated.

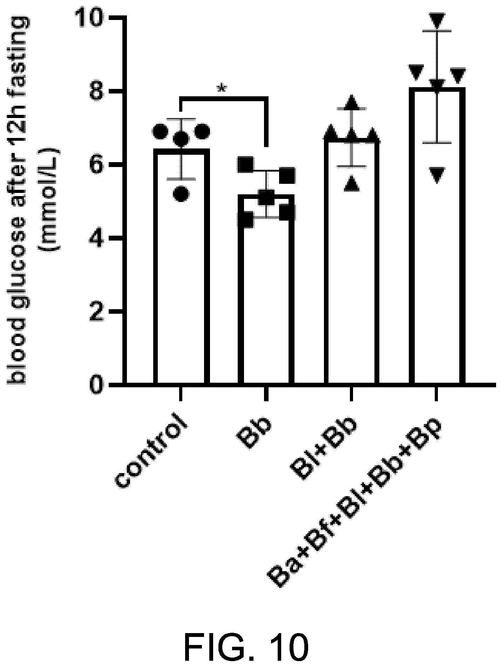

As shown in FIG. 10 for the result of fasting blood glucose level, gavage of *Bifidobacterium bifidum* ibiome001 (Bb) significantly reduced fasting blood glucose levels of high fat diet-induced obese and diabetic mice, while there is no significant improvement in fasting blood glucose levels of mice when given gavage composition containing other *Bifidobacterium spp..*

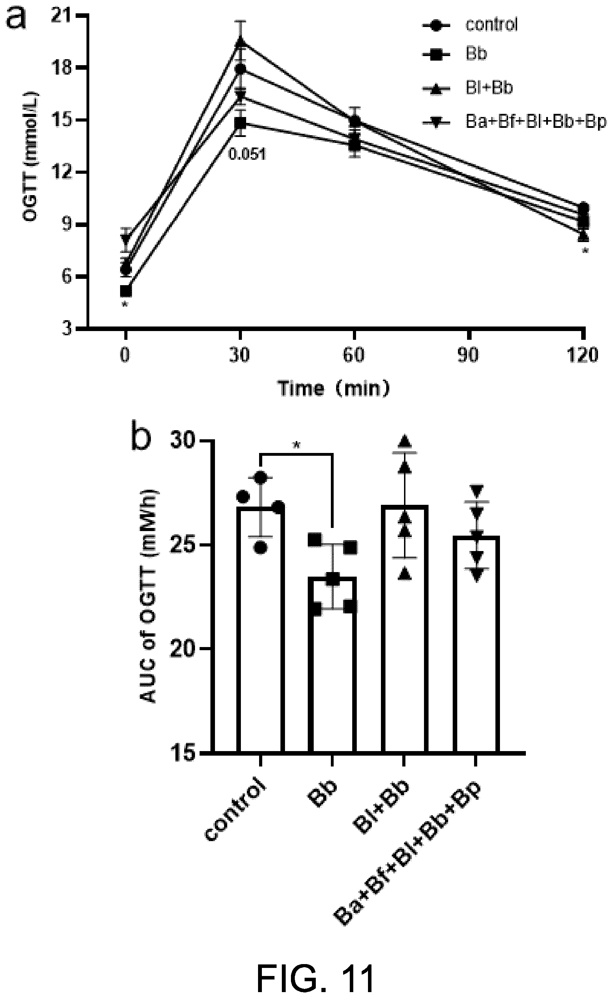

As shown in a of FIG. 11, Bb group has showed significant reduction of blood glucose levels since time point 30 min of OGTT (FIG. 11a, P=0.051) and showed significant reduction of area under OGTT curve (b of FIG. 11) compared to control group, while there is no significant improvement in real-time blood glucose levels or AUC of OGTT curve of mice when given gavage of composition containing other *Bifidobacterium spp..*

### 3. Effect on mouse adipose tissue weight

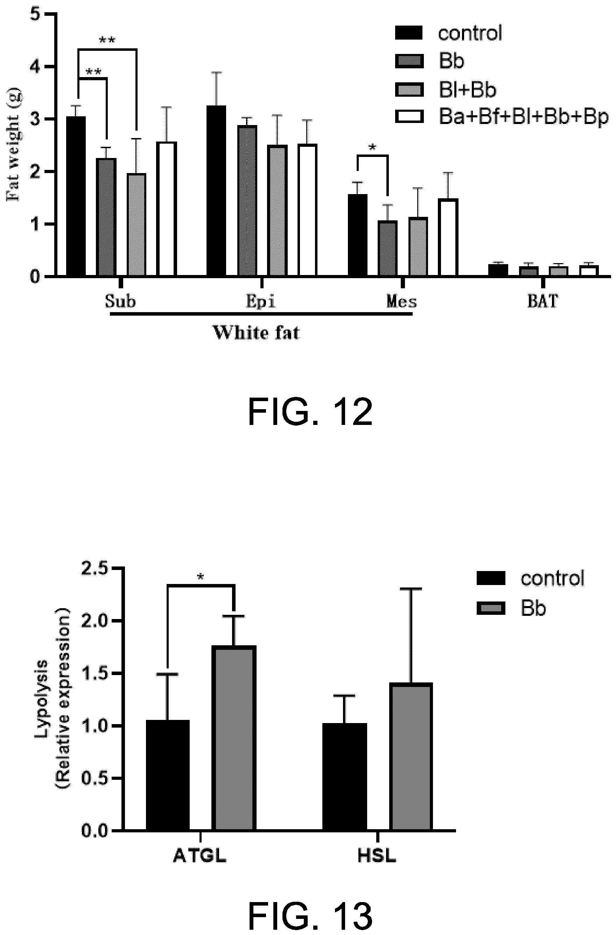

At the end of week 13, all groups of mice were euthanized after 12-hour fasting for plasma and adipose tissue (mesenteric white adipose tissue (WAT), subcutaneous WAT, epididymal WAT and interscapular brown adipose tissue (iBAT)) collection. All adipose tissue samples were weighed, and results were recorded and plotted in FIG. 12. It has been shown that *Bifidobacterium bifidum* ibiome001 (Bb) group shows significant reduction of weights of subcutaneous WAT and mesenteric WAT, while *Bifidobacterium bifidum* ibiome001 and *Bifidobacterium longum* (Bb+Bl) group only shows significant reduction of weight of subcutaneous WAT, and there are no significant changes of adipose tissue weights in 5-Mix group.

### 4. Effect on mouse lipolytic gene expression

RNAs of different types of white adipose tissue (WAT) described above were extracted for measuring expression of lipolysis related genes via qPCR method. Detailed procedures are described as follows:

Extraction of tissue RNA: tissues RNAs are extracted with an RNA extraction kit for animal tissues (purchased from TIANGEN BIOTECH (BEIJING) Co., Ltd., Cat. No.: DP424) under instruction of technical manual. RNA concentrations and RNA purity were measured by NanoDrop, and agarose gel electrophoresis.

Reverse transcription: add 2µL of 5× gDNA Buffer to 2µg of tissue total RNA, then add RNase-Free ddH2O up to a total volume of 10µL. Incubate at 42°C for 3 min after short centrifugation, then add 2µL of 10× Fast RT Buffer 2µL, 1µL of RT Enzyme Mix, 2µL of FQ-RT Primer Mix, and add ddH₂O up to a total volume of 20µL, after 42°C water bath for 15 minutes, react at 95°C for 3 minutes. Reverse transcription products are stored at -80°C.

qPCR amplification procedure: add 2µg of cDNA , 10µL SYBR dye, 0.8µL of corresponding primer, and add ddH₂O up to a total volume of 20µL. The reaction condition: denaturation at 95°C for 10 minutes, 40 cycles of amplification (95°C for 15 seconds, 60°C for 1 minute). There are 2 copies for every single reaction, and the results of relative quantitative analysis are calculated with 2^{-ΔΔCt}.

The results of qPCR is shown in FIG. 13. It has been shown that gavage of *Bifidobacterium bifidum* ibiome001 (Bb) significantly increases the expression of the lipolytic genes ATGL and HSL, in which there was significant difference in the degree of increment of ATGL (P < 0.05).

### 5. Effect of mouse glycated hemoglobin and plasma insulin

Given that the glycated hemoglobin level is a gold standard for assessing blood glucose regulation capacity, levels of glycated hemoglobin and insulin of plasma were measured with corresponding kit (purchased from CUSABIO TECHNOLOGY LLC; Cat. No.: CSB-E08141m and CSB-E05071m).

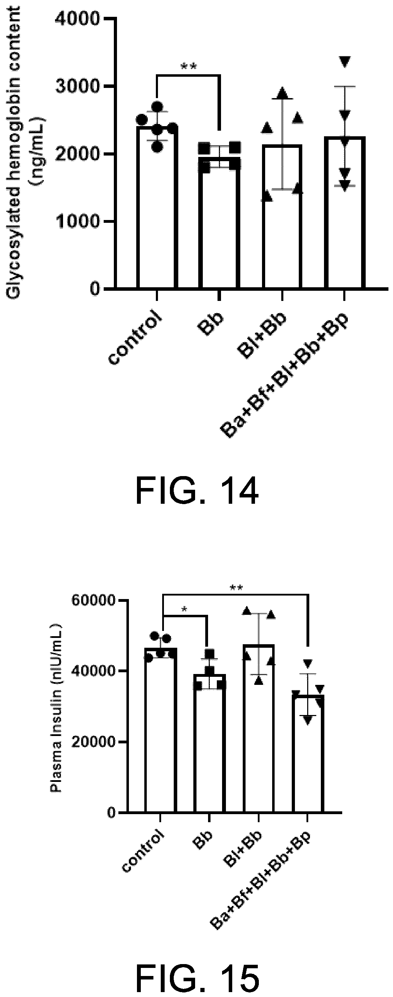

Glycated hemoglobin levels are the gold standard for measuring glycemic control. As shown in FIG. 14, gavage of *Bifidobacterium bifidum* ibiome001 (Bb) significantly lowered plasma glycated hemoglobin levels in high fat diet-induced obese and diabetic mice, while there were no significant changes in plasma glycated hemoglobin levels when given gavage of composition containing other *Bifidobacterium spp..*

As shown in FIG. 15, gavage of *Bifidobacterium bifidum* ibiome001 (Bb) significantly lowered plasma insulin levels of high fat diet-induced obese and diabetic mice, while there were no significant changes in plasma insulin levels when given gavage of composition containing *Bifidobacterium bifidum* ibiome001 and *Bifidobacterium longum* (Bb+BI).

In summary, gavage of *Bifidobacterium bifidum* ibiome001 (Bb) can significantly reduce body weight gain and white adipose tissue weight gain of high fat diet-induced obese and diabetic mice, and enhances expression of lipolytic genes ATGL and HSL. Also, the present invention significantly lowers fasting blood glucose levels, area under curve (AUC) of the diabetic mice oral glucose tolerance (OGTT) curve, plasma glycated hemoglobin levels and insulin levels. It is suggested that *Bifidobacterium bifidum* ibiome001 is a promising potential probiotic strain for the treatment of metabolic syndrome such as obesity, diabetes and the like, and has a significantly better effect than other *Bifidobacterium* strains/compositions.

Although the embodiments of the present invention have been described in detail, it will be obvious to those skilled in the art that the scope of the present invention is not limited to these embodiments and that various changes and modifications are possible without departing from the technical spirit of the present invention as defined in the appended claims. It should be within the protection scope defined by the Claims of the present invention.

### Industrial practicability

Gavage of *Bifidobacterium bifidum* ibiome001 can significantly reduce body weight and white adipose tissue weight of high-fat diet-induced obese diabetic mice, and enhances the expression of the lipolytic gene ATGL and HSL. Moreover, fasting glucose, oral glucose tolerance, plasma glycated hemoglobin and insulin levels have been significantly reduced. Thus, *Bifidobacterium bifidum ibiome001* is a potential functional strain for preventing and treating metabolic syndromes such as obesity, diabetes and the like, which is more effective than other *Bifidobacterium bifidum* strains or compositions, and it may have promising industrial practicability.

## Claims

1. A *Bifidobacterium bifidum* ibiome001, **characterized in that**, the *Bifidobacterium bifidum* strain was deposited at Guangdong Microorganism Strain Preservation Center, with an address of 5th Floor, Building 59, No. 100 Xianlie Central Road, Guangzhou City, Guangdong Province, Institute of Microbiology, Guangdong Academy of Sciences; a deposit date of May 17, 2022; and an accession number of GDMCC No. 62473.

2. A method for detecting *Bifidobacterium bifidum* ibiome001 according to Claim 1, **characterized in that**, the *Bifidobacterium bifidum* strain contains at least one specific gene segment or complementary segment of SEQ ID NO.2-5.

3. A composition, **characterized in that**, the composition comprises the *Bifidobacterium bifidum* ibiome001 according to Claim 1, and a pharmaceutically acceptable carrier.

4. The composition according to Claim 3, **characterized in that**, the pharmaceutically acceptable carrier comprises one, two or more of the following commonly used in medicine: filling agent, binding agent, wetting agent, disintegrating agent, lubricant, flavoring agent, diluent agent and absorption promoting agent.

5. Use of the *Bifidobacterium bifidum* ibiome001 according to Claim 1 or use of the composition according to Claim 3 or 4 in preparing functional microbial agents or pharmaceuticals, wherein the functional microbial agents or pharmaceuticals are used for preventing or treating one, two or more of the following diseases or symptoms (a) to (j) in mammals:
(a) diabetes;
(b) metabolic syndrome;
(c) abnormal glycated hemoglobin levels;
(d) abnormal insulin sensitivity;
(e) impaired fasting glucose;
(f) impaired glucose tolerance;
(g) abnormal fasting insulin levels;
(h) obesity;
(i) body weight gain; and
(j) increased adipose tissue weight.

6. Use of the *Bifidobacterium bifidum* ibiome001 according to Claim 1 or use of the composition according to Claim 3 or 4 in preparing functional microbial agents and pharmaceuticals for activating GPR120.

7. Use of the *Bifidobacterium bifidum* ibiome001 according to Claim 1 or use of the composition according to Claim 3 or 4 in preparing functional microbial agents and pharmaceuticals for improving the expression of the lipolytic gene ATGL and/or HSL.

8. The use according to Claim 5, **characterized in that**, the diabetes is type 2 diabetes.

9. The use according to Claim 5 or 8, **characterized in that**, the mammals are high-fat diet mammals.

10. Use of the *Bifidobacterium bifidum* ibiome001 according Claim 1 or use of the composition according to Claim 3 or 4 in preparing food or dietary supplement.
